# EUROPEAN PATENT APPLICATION

(11) **EP 4 190 270 A1**
(43) Date of publication of application: **07.06.2023**
(21) Application number: 21212292.3
(22) Date of filing: 03.12.2021
(51) Int. Cl.: A61B 90/00, A61B 1/267, A61B 1/00, G06T 7/13, G06N 3/02, G06T 7/73

(54) **ENDOSCOPE IMAGE PROCESSING DEVICE**

(71) Applicant: Ambu A/S, 2750 Ballerup (DK)
(72) Inventor: ALONSO DÍAZ, Alejandro, 2750 Ballerup (DK); GADE, Josefine Dam, 1973 Frederiksberg (DK); JØRGENSEN, Andreas Härstedt, 2610 Rødovre (DK); LASSEN, Lee Herluf Lund, 2760 Måløv (DK); YU, Dana Marie, 2750 Ballerup (DK)
(74) Representative: AWA Denmark A/S

(57) **Abstract**

Disclosed is an image processing device for estimating a quality measure of a bronchoscopy procedure performed using an endoscope. The endoscope comprising an image capturing device, the image processing device comprising a processing unit operationally connectable to the image capturing device. The processing unit is configured to: obtain an image captured by the image capturing device of the endoscope; process the image to estimate the location of one or more lumens in the image, and determine a quality measure of the bronchoscopy based on the estimated location of the one or more lumens.

## Description

### Field

The present disclosure relates to an image processing device, a display unit, an endoscope system, a method for estimating a quality measure of a bronchoscopy procedure, and a computer program product.

### Background

Endoscopes are widely used in hospitals for visually examining body cavities and obtaining samples of tissue identified as potentially pathological. An endoscope typically comprises an image capturing device arranged at the distal end of the endoscope either looking forward or to the side. An endoscope is further typically provided with a working channel allowing a medical device such as a gripping device, a suction device, or a catheter to be introduced.

A bronchoscopy is an example of an important endoscopic procedure. During a bronchoscopy procedure an endoscope is advanced through the bronchial tree. This may however be a challenging task as parts of the bronchial tree is very narrow providing little space for the endoscope. Unintentionally colliding the endoscope with the bronchial wall may be very unpleasant for the patient and should be avoided.

It is therefore important that new medical personnel are properly trained. For this purpose, phantom models of the bronchial tree have been developed allowing new medical personnel to train without risking inflicting harm to a patient.

Training on a phantom model of the bronchial tree is however not optimal as the phantom model presents an idealized case with no tissue movement. Furthermore, the pressure of a true clinical setting cannot be fully recreated. Furthermore, it may be difficult for the new medical personnel or trainers of the new medical personnel to properly evaluate their performance both when training on a phantom model and when training on real patients.

Thus, it remains a problem to provide an improved device / method for allowing medical personnel to train and / or evaluate their performance.

### Summary

According to a first aspect, the present disclosure relates to an image processing device for estimating a quality measure of a bronchoscopy procedure performed using an endoscope, the endoscope comprising an image capturing device, the image processing device comprising a processing unit operationally connectable to the image capturing device, wherein the processing unit is configured to:
obtain an image captured by the image capturing device of the endoscope;
process the image to estimate the location of one or more lumens in the image, and
determine a quality measure of the bronchoscopy based on the estimated location of the one or more lumens.

When the endoscope navigates through the bronchial tree a lumen should normally be located centrally in the image to lower the risk that the endoscope collides with the walls of the bronchial tree. Consequently, by using an estimated location of a lumen a simple and effective way of estimating a quality measure is provided.

The processing unit of the image processing device may be any processing unit, such as a central processing unit (CPU), a graphics processing unit (GPU), a microcontroller unit (MCU), a field-programmable gate array (FPGA), or any combination thereof. The processing unit may comprise one or more physical processors and/or may be combined by a plurality of individual processing units.

The image processing device may comprise a display, i.e. the image processing device may be integrated in a display unit. Alternatively / additionally, the image processing device may be operationally connectable to a separate display unit.

The processing unit may be configured to detect the lumen using an adaptive method such as a machine learning data architecture.

Alternatively, the processing unit may be configured to detect the lumen using a non-adaptive method relying on static rules. As an example, the processing unit may be configured to detect a lumen by using a method comprising: finding a set of connected pixels having an intensity value below a first threshold. The first threshold may be an absolute threshold, or a threshold determined based on the average intensity in an image. A group of connected pixels may be a set of pixels, where each pixel shares an edge or a corner with at least one other pixel in the set of pixels. A lumen may be detected if a set of connected pixels is found having a size above a second threshold.

The quality measure may be presented to the user during the procedure e.g. on a display. Alternatively, the quality measure may be presented to the user after the procedure has been completed and / or stored in a memory unit connected to the processing unit of the image processing device.

In some embodiments, the processing unit is configured to estimate the location of the one or more lumens by providing the image to a machine learning data architecture trained to identify the location of lumens in endoscope images.

Consequently, an effective and reliable way of determining positions of lumens is provided.

The machine learning data architecture may be a supervised machine learning architecture. The machine learning data architecture may be trained by obtaining a training data set comprising endoscope images of different parts of different bronchial trees and for each endoscope image having a human operator identify a lumen (if present) e.g. a human operator may for each image firstly specify if a lumen can be seen in the image (if the image capturing device is pointing towards the wall of the bronchial tree no lumen is visible), and secondly the location of the lumen. The location of the lumen may be a center of the lumen or a circumference of the lumen. The circumference of the lumen may be a circle e.g. the smallest circle that encompasses the lumen or the largest circle that can be fully contained within the lumen. Alternatively, the circumference may be a curve substantially arranged above the border of the lumen. The processing unit may implement the machine learning data architecture, i.e. the processing unit may provide the image to the machine learning data architecture by processing the image using the machine learning data architecture. Alternatively, the processing unit may provide the image, a parameterized version of the image, or a dimensionally reduced version of the image to another processing unit e.g. outside of the image processing device, where said another processing unit implements the machine learning data architecture.

In some embodiments, the machine learning data architecture is an artificial neural network such as a deep structured learning architecture. The machine learning data architectures may be U-Net (reference: Ronneberger et el. "U-Net: Convolutional Networks for Biomedical Image Segmentation" (2015), arXiv:1505.04597 [cs.CV] or Mask R-CNN (reference: He et al. "Mask R-CNN" (2017), arXiv:1703.06870 [cs.CV].

In some embodiments, the processing unit is configured to obtain a stream of images captured by the image capturing device of the endoscope, process the stream of images to estimate the location of the one or more lumens in the stream of images, and determine the quality measure of the bronchoscopy based on the estimated location of the one or more lumens in the stream of images.

Consequently, the quality measure may represent the quality of an entire procedure.

In some embodiments, the processing unit is further configured to obtain an optimum lumen location in the stream of images, and determine the quality measure based on both the optimum lumen location and the estimated location of the one or more lumens in the stream of images.

While it may generally be desirable to have a lumen arranged centrally, there may be situations where a lumen should not be arranged centrally e.g. if two lumens are present, then it may be desirable that each lumen is arranged with an offset to the center of image. Thus, by obtaining an optimum lumen location a more precise quality measure me be determined.

The optimum lumen location may be a zone of the image e.g. a zone centered in the center of image. The zone may have a circular or elliptical shape. Alternatively, the optimum lumen location may be a point in the image indicating the optimum location of a point of the lumen e.g. the center of the lumen. A value of the quality measure indicating a high quality may result if the location of the one or more lumens are close to the optimum lumen location and a value of the quality measure indicating a low quality may result if the one or more lumens are far away from the optimum lumen location. The quality measure may be based on a numerical number e.g. a number between 0 and 10. A sub quality measure may be determined for each image of the stream of images, wherein the quality measure is an average of the sub quality measures. The average may be a weighted average or an unweighted average.

In some embodiments, the processing unit is configured to continuously obtain optimum lumen locations.

Consequently, different optimal lumen locations may be obtained as the endoscope propagates through the airways.

In some embodiments, the processing unit is configured to estimate the location of the endoscope or the type of location in the bronchial tree and based on the estimated location or type of location is configured to determine optimum lumen locations.

The location may specify a particular part of the bronchial tree e.g. a particular branch of the bronchial tree. The location of the endoscope may be determined using any method such as magnetic tracking, image-based techniques such as machine learning methods e.g. as disclosed in PCT/EP2021/064985.

As an example, the bronchial tree may be divided into a number of sections and for each section optimum lumen location(s) may be specified, the processing unit may then estimate in which section of the bronchial tree the endoscope is present and retrieve the corresponding optimum lumen locations). The optimum lumen locations may be provided by an experienced medical professional and stored in a memory unit e.g. a memory unit of the image processing device.

The type of location in the bronchial tree may specify if a single lumen or more than a single lumen is present e.g. if more than one lumen is present optimum lumen location may be enlarged.

In some embodiments, the processing unit is further configured to estimate the operational state of the endoscope, the operational state indicates if the endoscope is navigating through the bronchial tree or investigating an area of the bronchial tree and wherein the processing unit is configured to determine the quality measure based on both the estimated location of the one or more lumens and the estimated operational state of the endoscope.

Consequently, a more precise quality measure may be determined.

In some embodiments, the processing unit is configured to estimate the operational state of the endoscope by processing the image or stream of images using a machine learning data architecture trained to determine based on endoscope images or streams of images if the operator is navigating through the bronchial tree or investigating an area of the bronchial tree.

Consequently, an effective way of determining operational state is provided.

The machine learning data architecture may be a supervised machine learning architecture. The machine learning data architecture may be trained by obtaining a training data set comprising endoscope images from different bronchoscopy procedures and for each endoscope image having a human operator identify if the current image is from a part of the bronchoscopy procedure where the medical professional is navigating through the bronchial tree or investigating an area of the bronchial tree.

In some embodiments, the requirements for the location of the one or more lumens are more strict if it is estimated that the endoscope is navigating through the bronchial tree than if it is estimated that the endoscope is investigating an area of the bronchial tree.

As an example, there may be no requirements to the location of a lumen if it is estimated that the endoscope is investigating an area of the bronchial tree.

According to a second aspect, the present disclosure relates to a display unit for displaying images obtained by an image capturing device of an endoscope, wherein the display unit comprises an image processing device as disclosed in relation to the first aspect of the present disclosure.

According to a third aspect, the present disclosure relates to an endoscope system comprising an endoscope and an image processing device as disclosed in relation to the first aspect of the present disclosure,
wherein the endoscope has an image capturing device and the processing unit of the image processing device is operationally connectable to the image capturing device of the endoscope.

In some embodiments, the image processing device forms part of a display unit as disclosed in relation to the second aspect of the disclosure.

According to a fourth aspect, the present disclosure relates to
a method for estimating a quality measure of a bronchoscopy procedure based on an image captured by an image capturing device of an endoscope, wherein the method comprises the steps of:
obtain an image captured by the image capturing device of the endoscope;
process the image to estimate the location of one or more lumens in the stream of images,
determine a quality measure of the bronchoscopy based on the estimated location of the one or more lumens.

According to a fifth aspect, the present disclosure relates to a computer program product comprising program code means adapted to cause a data processing system to perform the steps of the method as disclosed in relation to the fourth aspect of the present disclosure, when said program code means are executed on the data processing system.

In some embodiments, said computer program product comprises a non-transitory computer-readable medium having stored thereon the program code means.

The different aspects of the present disclosure can be implemented in different ways including image processing devices, display units, endoscope systems, methods for estimating a quality measure, and computer program products described above and in the following, each yielding one or more of the benefits and advantages described in connection with at least one of the aspects described above, and each having one or more preferred embodiments corresponding to the preferred embodiments described in connection with at least one of the aspects described above and/or disclosed in the dependent claims. Furthermore, it will be appreciated that embodiments described in connection with one of the aspects described herein may equally be applied to the other aspects.

### Brief description of the drawings

The above and/or additional objects, features and advantages of the present disclosure, will be further elucidated by the following illustrative and non-limiting detailed description of embodiments of the present disclosure, with reference to the appended drawings, wherein:
Fig. 1 shows an example of an endoscope.
Fig. 2 shows an example of a display unit that can be connected to the endoscope shown in Fig. 1.
Fig. 3 shows a schematic drawing of an image processing device according to an embodiment of the disclosure.
Fig. 4 shows a flowchart of a method for estimating a quality measure of a bronchoscopy according to an embodiment of the disclosure.
Fig. 5a-c show schematically endoscope views.
Fig. 6 illustrate how a machine learning data architecture may be trained to estimate locations of one or more lumens in endoscope images.
Fig. 7a-b illustrates how one or more lumen may be identified in endoscope images according to an embodiment of the disclosure.

### Detailed description

In the following description, reference is made to the accompanying figures, which show by way of illustration how the embodiments of the present disclosure may be practiced.

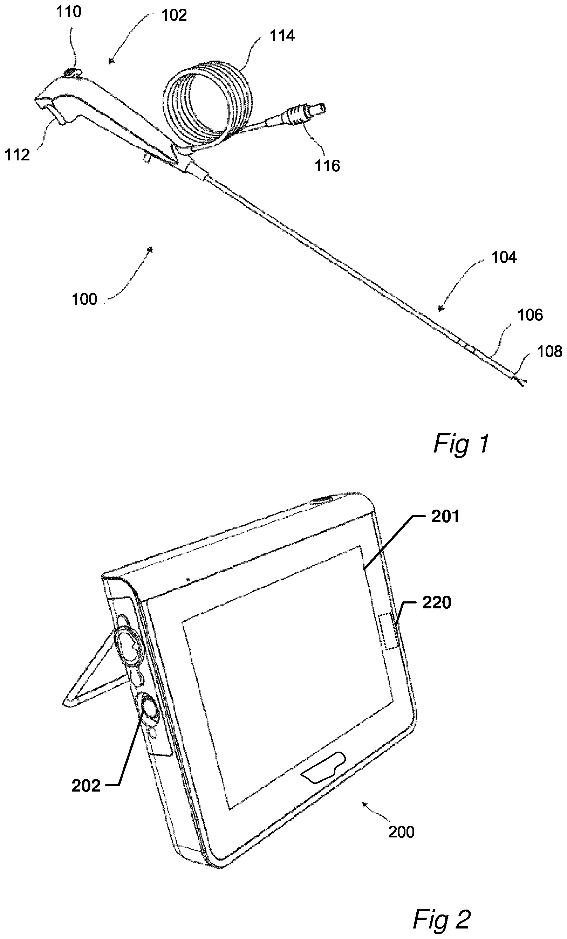

Fig. 1 illustrates an example of an endoscope 100. This endoscope may be adapted for single-use. The endoscope 100 is provided with a handle 102 attached to an insertion tube 104 provided with a bending section 106. The endoscope 100 may also be designed without a bending section 106. The illustrated endoscope 100 is a flexible endoscope with a flexible insertion tube 104, but the principle of the disclosure can also be used for other types of endoscopes, such as laryngoscopes or laparoscopes.

The insertion tube 104 as well as the bending section 106 may be provided with one or several working channels such that tools or instruments, such as a gripping device or a catheter, may extend from the tip and be inserted into a human body via the endoscope. One or several exit holes of the one or several channels may be provided in a tip part 108 of the endoscope 100. In addition to the exit holes, a camera sensor, such as a CMOS sensor or any other image capturing device, as well as one or several light sources, such as light emitting diodes (LEDs), fiber, or any other light emitting devices, may be placed in the tip part 108. By having the camera sensor and the light sources and a display unit 200, illustrated in Fig. 2, configured to display images based on image data captured by the camera sensor, an operator is able to see and analyse an inside of the human body in order to for instance localize a position for taking a sample. In addition, the operator will be able to control the instrument in a precise manner due to the provided visual feedback. Further, since some diseases or health issues may result in a shift in natural colours or other visual symptoms, the operator is provided with valuable input for making a diagnosis based on the image data provided via the image capturing device and the display unit 200. The display unit comprises a display 201, a processing unit 220 (only schematically shown), and a connection port 202 for operationally connecting the endoscope to the processing unit 220. The connection port 202 may further be used to provide power to the endoscope.

In order to make it possible for the operator to direct the camera sensor such that different field of views can be achieved, the endoscope has a bending section 106 that can be bent in different directions with respect to the insertion tube 104. The bending section 106 may be controlled by the operator by using a knob 110 placed on the handle 102. The handle 102 illustrated in Fig. 1 is designed such that the knob 110 is controlled by a thumb of the operator, but other designs are also possible. In order to control a tool, e.g. a gripping device or other device provided via a working channel, a push button 112 may be used. When the tool is not built as part of the endoscope, a push button will be arranged at a proximal end of the tool outside the handle 102. The handle 102 illustrated in Fig. 1 is designed such that a index finger of the operator is used for controlling the gripping device, but other designs are also possible.

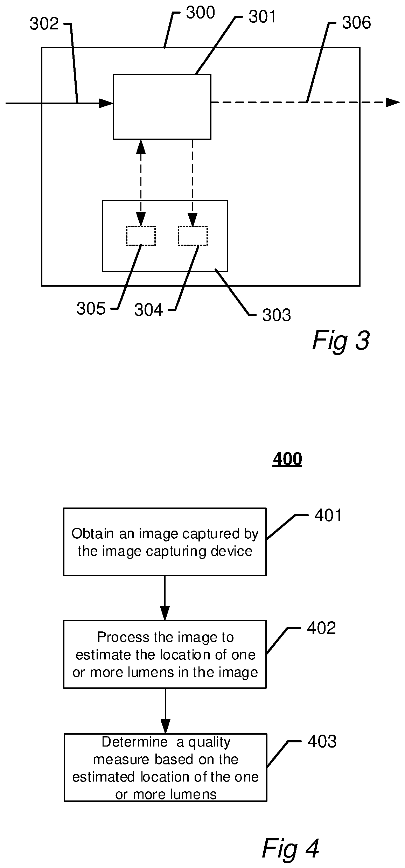

Fig. 3 show a schematic drawing of an image processing device 300 for estimating a quality measure of a bronchoscopy procedure performed using an endoscope (not shown) according to an embodiment of the disclosure. The endoscope comprises an image capturing device. The image processing device 300 comprises a processing unit 301 operationally connectable 302 to the image capturing device. The processing unit 301 is configured to obtain an image captured by the image capturing device of the endoscope. The processing unit may be wired connectable to the image capturing device. As an example, the image processing device may comprise a connection port for operationally connecting the endoscope to the processing unit 301. The connection port may further be used to provide power to the endoscope. Alternatively, the processing unit 301 may be wireless connectable to the image capturing device e.g. the image processing device 300 may comprise a wireless communication unit (not shown) configured to receive images from the image capturing device. The processing unit 301 is further configured to process the image to estimate the location of one or more lumens in the image, and determine a quality measure 304 of the bronchoscopy based on the estimated location of the one or more lumens. The processing unit may be configured to estimate the location of the one or more lumens by processing the image using a machine learning data architecture 305 trained to identify the location of lumens in endoscope images. As an example, the machine learning data architecture may be stored in a memory unit 303 of the image processing device. The quality measure 304 may be stored in the memory unit 303. Alternatively / additionally, the quality measure may be provided 306 to a display (not) shown to be displayed to an operator, possibly together with endoscope images.

Fig. 4 shows a flowchart of a method 400 of a method for estimating a quality measure of a bronchoscopy procedure based on an image captured by an image capturing device of an endoscope. In step 401 an image captured by the image capturing device of the endoscope is obtained. Next, in step 402 the image is processed to estimate the location of one or more lumens in the stream of images. Then, in step 403, a quality measure of the bronchoscopy is determined based on the estimated location of the one or more lumens.

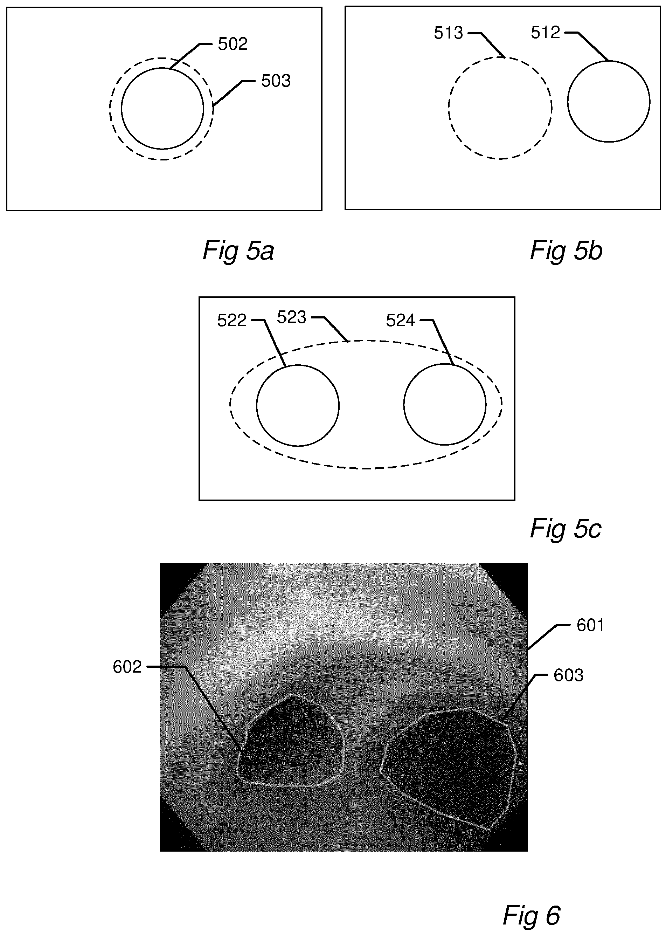

Fig. 5a-c show schematically images captured by an image capturing device of an endoscope according to an embodiment of the present disclosure. In Fig. 5a and Fig. 5b is a single lumen 502 512 shown and in Fig. 5c is two lumens shown 522 524. In Fig. 5a is further shown an optimum lumen location 503. The optimum lumen location 503 is for this image arranged centrally. Thus, at least when the endoscope is navigating through the bronchial tree, the lumen should be arranged centrally to lower the risk that the endoscope distal tip is colliding with the bronchial wall. The optimum lumen location 503 may not be shown to the medical professional operating the endoscope i.e. the optimum lumen location 503 may only be available to an image processing device for determining a quality measure e.g. an image processing device as disclosed in relation to Fig. 3 as explained previously.

However, the optimum lumen location 503 may be shown to the medical professional. As an example, if the images captured by the image capturing device are displayed on a display unit, then the optimum lumen location 503 may be indicated on the display unit e.g. it may be overlaid the endoscope images. The processing unit of the image processing device may be configured to estimate the operational state of the endoscope by processing the image or a stream of images using a machine learning data architecture trained to determine, based on endoscope images or streams of images, if the operator is navigating through the bronchial tree or investigating an area of the bronchial tree. Based on the estimated operational state, the optimal lumen location 503 may or may not be shown to the medical professional e.g. optimal lumen location 503 may only be displayed, when it is estimated that the operator is navigating through the bronchial tree. This may be especially useful for training new medical professionals. Furthermore, by selectively displaying the optimum lumen location, the medical professional may not be distracted when investigating an area of the bronchial tree e.g. for a pathological condition.

Fig. 5a shows a typical view of the image capturing device when the endoscope is arranged in a part of the bronchial tree where no branching is present In Fig. 5a, the lumen 502 is arranged fully within the optimum lumen location 503. In this situation a quality measure indicating a high quality may result.

Fig. 5b shows a situation where the lumen 512 is arranged fully outside the optimum lumen location 513. In this situation a quality measure indicating a low quality may result.

Fig. 5c shows a typical view of the image capturing device when the endoscope is arranged in a part of the bronchial tree where a branching is present. In Fig. 5c, the lumens 522 and 524 are arranged fully within the optimum lumen location 523. In this situation a quality measure indicating a high quality may result. In Fig. 5c it can further be seen that the optimum lumen location 523 has been enlarged.

Fig. 6 illustrates how a machine learning data architecture may be trained to estimate locations of one or more lumens in endoscope images. Shown is a single image 601 of a training data set. A typical training data set comprises endoscope images of different parts of different bronchial trees, i.e. endoscope images from a significant number of endoscope examinations. To train the machine learning data architecture a human operator indicates the location of lumens, in this concrete example two lumens 602 603. The location of the lumens is indicated by drawing up the circumference of each lumen 602 603.

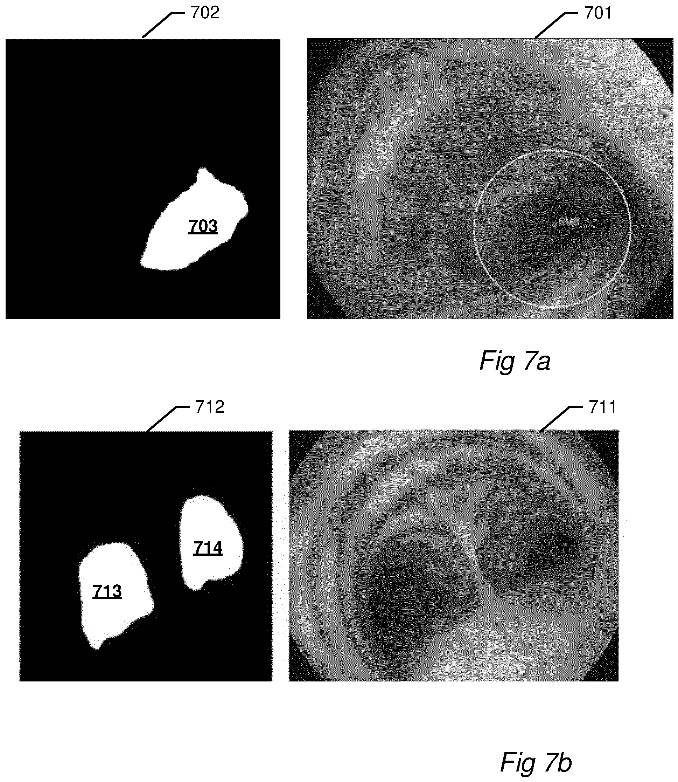

Fig. 7a-b illustrates how one or more lumen may be identified in endoscope images according to an embodiment of the disclosure. The one or more lumens are identified using a machine learning data architecture trained to estimate locations of one or more lumens in endoscope images as disclosed in relation to Fig. 6. In Fig. 7a, the input image 701 is shown to the right and the output from the machine learning data architecture 702 is shown to the left. As the machine learning data architecture has been trained to estimate the circumference of lumen, not only the location of the lumen 703 is estimated but also the circumference of the lumen 703. Fig. 7b shows another example, where the input image 711 is shown to the right and the output from the machine learning data architecture 712 is shown to the left. In this example, the location of two lumens 713 714 are estimated.

Although some embodiments have been described and shown in detail, the invention is not restricted to them, but may also be embodied in other ways within the scope of the subject matter defined in the following claims. In particular, it is to be understood that other embodiments may be utilised and structural and functional modifications may be made without departing from the scope of the present invention.

In device claims enumerating several means, several of these means can be embodied by one and the same item of hardware. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, integers, steps or components but does not preclude the presence or addition of one or more other features, integers, steps, components or groups thereof.

## Claims

1. An image processing device for estimating a quality measure of a bronchoscopy procedure performed using an endoscope, the endoscope comprising an image capturing device, the image processing device comprising a processing unit operationally connectable to the image capturing device, wherein the processing unit is configured to:
obtain an image captured by the image capturing device of the endoscope;
process the image to estimate the location of one or more lumens in the image, and
determine a quality measure of the bronchoscopy based on the estimated location of the one or more lumens.

2. An image processing device according to claim 1, wherein the processing unit is configured to estimate the location of the one or more lumens by providing the image to a machine learning data architecture trained to identify the location of lumens in endoscope images.

3. An image processing device according to claims 1 or 2, the processing unit is configured to obtain a stream of images captured by the image capturing device of the endoscope, process the stream of images to estimate the location of the one or more lumens in the stream of images, and determine the quality measure of the bronchoscopy based on the estimated location of the one or more lumens in the stream of images.

4. An image processing device according to claim 3, wherein the processing unit is further configured to obtain an optimum lumen location in the stream of images, and determine the quality measure based on both the optimum lumen location and the estimated location of the one or more lumens in the stream of images.

5. An image processing device according to claim 4, wherein the processing unit is configured to continuously obtain optimum lumen locations.

6. An image processing device according to claim 5, wherein the processing unit is configured to estimate the location of the endoscope or the type of location in the bronchial tree and based on the estimated location or type of location is configured to determine optimum lumen locations.

7. An image processing device according to any one of claims 1 to 6, wherein the processing unit is further configured to estimate the operational state of the endoscope, the operational state indicates if the endoscope is navigating through the bronchial tree or investigating an area of the bronchial tree and wherein the processing unit is configured to determine the quality measure based on both the estimated location of the one or more lumens and the estimated operational state of the endoscope.

8. An image processing device according to claim 7, wherein the processing unit is configured to estimate the operational state of the endoscope by processing the image or stream of images using a machine learning data architecture trained to determine based on endoscope images or streams of images if the operator is navigating through the bronchial tree or investigating an area of the bronchial tree.

9. An image processing device according to claims 7 or 8, wherein the requirements for the location of the one or more lumens are more strict if it is estimated that the endoscope is navigating through the bronchial tree than if it is estimated that the endoscope is investigating an area of the bronchial tree.

10. A display unit for displaying images obtained by an image capturing device of an endoscope, wherein the display unit comprises an image processing device according to any one of claims 1 to 9.

11. An endoscope system comprising an endoscope and an image processing device according to any one of claims 1 to 9, wherein the endoscope has an image capturing device and the processing unit of the image processing device is operationally connectable to the image capturing device of the endoscope.

12. An endoscope system according to claim 11, wherein the image processing device forms part of a display unit according to claim 10.

13. A method for estimating a quality measure of a bronchoscopy procedure based on an image captured by an image capturing device of an endoscope, wherein the method comprises the steps of:
obtain an image captured by the image capturing device of the endoscope;
process the image to estimate the location of one or more lumens in the stream of images,
determine a quality measure of the bronchoscopy based on the estimated location of the one or more lumens.

14. A computer program product comprising program code means adapted to cause a data processing system to perform the steps of the method according to claim 13, when said program code means are executed on the data processing system.

15. A computer program product according to claim 14, wherein said computer program product comprises a non-transitory computer-readable medium having stored thereon the program code means.
